# EUROPEAN PATENT APPLICATION

(11) **EP 3 536 312 A1**
(43) Date of publication of application: **11.09.2019**
(21) Application number: 18160647.6
(22) Date of filing: 08.03.2018
(51) Int. Cl.: A61K 9/50, A61K 38/00, A61K 39/00

(54) **A METHOD FOR PRODUCING ACID RESISTANT MACROBEADS CONTAINING NANOPARTICLES AND USE OF THE MACROBEADS FOR ELICITING AN IMMUNE RESPONSE**

(71) Applicant: Erber Aktiengesellschaft, 3131 Getzersdorf bei Traismauer (AT)
(72) Inventor: Nilubol, Dachrit, 11000 Nonthaburi (TH); Tantituvanont, Angkana, 11000 Nonthaburi (TH)
(74) Representative: Schiweck Weinzierl Koch Patentanwälte Partnerschaft mbB

(57) **Abstract**

The present invention relates to methods and means for the production of alginate/chitosan acid-resistant macrobeads comprising alginate/chitosan nanoparticles, wherein the nanoparticles comprise a bioactive agent, wherein the bioactive agent is associated via physicochemical interaction with a polymer. The invention further relates to nanoparticles and/or macrobeads obtained by methods of the invention and such nanoparticles and/or macrobeads for use in eliciting an immune response in an animal. Also encompassed by the invention is the use of a porcine epidemic diarrhea virus (PEDV) antigen as defined herein for producing a nanoparticle or acid-resistant macrobeads and a vaccine comprising such an antigen.

SEQUENCE LISTING

<110>  ERBER AG

<120>  A METHOD FOR PRODUCING ACID RESISTANT MACROBEADS CONTAINING
       NANOPARTICLES AND USE OF THE MACROBEADS FOR ELICITING AN IMMUNE
       RESPONSE

<130>  ERB15956EP

<160>  4

<170>  PatentIn version 3.5

<210>  1
<211>  24
<212>  DNA
<213>  Artificial Sequence

<220>
<223>  Primer for amplication of PEDV spike gene

<400>  1
gccatggctg tttattctgt tacg                                              24


<210>  2
<211>  42
<212>  DNA
<213>  Artificial sequence

<220>
<223>  Primer for amplication of PEDV spike gene

<400>  2
ttagtgatgg tgatggtgat gtgggttctt acatgctgca gt                          42


<210>  3
<211>  29
<212>  DNA
<213>  Artificial sequence

<220>
<223>  Primer for amplication of PEDV spike gene

<400>  3
ctgggatccg tgctgtttat tctgttacg                                         29


<210>  4
<211>  52
<212>  DNA
<213>  Artificial sequence

<220>
<223>  Primer for amplication of PEDV spike gene

<400>  4
ctagtcgact cagtgatggt gatggtgatg tgggttctta catgctgcag tg               52

## Description

### TECHNICAL FIELD OF THE INVENTION

The present invention relates to methods and means for the production of alginate acid-resistant macrobeads comprising alginate/chitosan nanoparticles, wherein the nanoparticles comprise a bioactive agent, wherein the bioactive agent is associated via physicochemical interaction with a polymer. The invention further relates to nanoparticles and/or macrobeads obtained by methods of the invention and such nanoparticles and/or macrobeads for use in eliciting an immune response in an animal. Also encompassed by the invention is the use of a porcine epidemic diarrhea virus (PEDV) antigen as defined herein for producing a nanoparticle or acid-resistant macrobeads and a vaccine comprising such an antigen.

### BACKGROUND ART

The delivery of bioactive agents, such as DNA or proteins, via the stomach into the gut and optionally subsequently into the blood is hampered by the conditions in the stomach, which include an acidic environment and the presence of proteases or nucleases. These conditions provide for the disassembly or destruction of proteins and nucleic acids before they can be absorbed in the gut. It is thus necessary that these bioactive agents are protected against degradation. One possible way to improve the uptake of proteins or nucleic acids in the gastrointestinal tract is to encapsulate them in colloidal nanoparticles that protect the bioactive agent from being degraded and facilitate their transportation into systemic circulation.

An example of a bioactive agent is porcine epidemic diarrhea virus (PEDV). PEDV, a RNA virus in the *Coronaviridae* family, is a causative agent of porcine epidemic diarrhea (PED). PED is a devastating enteric disease characterized by vomiting and severe watery diarrhea leading to death. Pigs at all ages are susceptible to PED, but high mortality is observed in pigs younger than a week of age. Infected pigs that are less than one week old have mortality rate up to 100%.

To successfully control PEDV during the outbreak, planned exposure of sows with minced intestines of PEDV-infected pigs has long been suggested. However, side effects of this management protocol, including an increased percentage of mummified fetuses and risks of contamination by other pathogens, have been observed. To prevent herds from a re-infection of PED, intramuscular vaccination with either modified live or killed PEDV vaccine has been implemented as an alternative tool. However, attempts to intramuscularly immunize pigs with either modified live and killed PEDV vaccine have shown varying degrees of success. Herds with intensive use of PEDV vaccine still experienced PED outbreaks. The route of vaccination has contributed to the unsuccessful results. Because mucosal immunity, especially secretory Immunoglobulin A (slgA), plays an important role in controlling PEDV infection, intramuscular vaccination with PEDV vaccines is not completely effective and does not induce mucosal immunity. Due to the facts that an oral PEDV vaccine is not commercially available, farmers have tried stimulating the mucosal immunity by orally administrating sows with minced intestines of PEDV-infected pigs at pre-farrow. However, the method also gains a varying degree of success. The reasons are due to the following: The dosage of PEDV in minced intestines of infected pigs administered into sows to successfully induce mucosal immune response is insufficient and varying. In addition, there are risks of contamination by other harmful pathogens and the introduction of live virus in herd is a risk to cause a PED outbreak.

So far, some nanoparticulate oral delivery systems have been used for oral vaccination. For example, polyethyleneimine (PEI) has been used to deliver plasmid DNA into immune cells. PEI contains primary amine that can interact with the phosphate group of plasmid DNA to form a complex called polyplex that can enhance the uptake of plasmid DNA into the cell by endocytosis. Though PEI has high efficiency in delivering plasmid DNA into cells, the application of PEI is hampered by its cytotoxicity and non-specific binding to the serum protein.

An alternative method is the preparation of polyplexes coated by alginate. This method will reduce the net charge, therefore reducing the cytotoxicity and enhancing the delivery of plasmid DNA into cell in the presence of serum. However, the above system cannot protect the plasmid DNA from degradation in the gut when given orally. Another method is to prepare calcium alginate nanoparticles to prevent plasmid DNA degradation in the gut. For a better acid protection, chitosan may be added. Chitosan also functions as a mucoadhesive agent, which will increase the retention time of calcium alginate nanoparticles, subsequently increase the uptake by the immune cells. Although calcium alginate nanoparticles with chitosan can protect the degradation of plasmid DNA by the acidic environment in the gut, the efficiency of the nanoparticles in delivering plasmid DNA into the immune cells is not as efficient as the polyplex.

Li et al. (2007), Journal of Biomedical Materials research, 83A(2):383-390 disclose chitosan/alginate nanoparticles that include BSA as exemplary protein for oral delivery. Machado et al. (2013), Langmuir, 29(51):15926-15935 disclose the encapsulation of DNA in alginate nanoparticles but not the additional use of a cationic polymer or chitosan. Jian et al. (2007), Biotechnology and Bioprocess Engineering, 12:684-689 disclose the use of DNA/PEI/Alginate polyplexes as delivery system. Hence, alginate/chitosan nanoparticles comprising DNA and a cationic polymer like e.g. PEI have not yet been described.

There is still a need for alternative oral delivery of bioactive agents through an encapsulation platform, protecting the bioactive agent in the stomach and releasing it in the gut. The technical problem therefor is to comply with this need.

### SUMMARY OF THE INVENTION

The technical problem is solved by the claimed subject-matter. The invention provides a method for producing acid resistant macrobeads containing nanoparticles, wherein the nanoparticles comprise a bioactive agent, wherein the bioactive agent is associated via physicochemical interaction with a polymer. Further, the invention relates to a primer set for amplifying the PEDV spike gene, chitosan/alginate nanoparticles, acid-resistant alginate macrobeads and a PEDV antigen. In addition, the invention provides the use of a PEDV antigen for producing a chitosan/alginate nanoparticle or acid resistant macrobead.

According to a first aspect the invention provides a method for producing acid resistant macrobeads containing nanoparticles, wherein the nanoparticles comprise a bioactive agent, wherein the bioactive agent is associated via physicochemical interaction with a polymer, the method comprising:
(a) mixing a solution comprising the bioactive agent that is associated via physicochemical interaction with a polymer with a first alginate solution to obtain an alginate/bioactive agent mixture
(b) mixing a first calcium chloride solution with a first chitosan solution to obtain a first calcium chloride/chitosan solution and
(c) adding the first calcium chloride/chitosan solution obtained in step (b) to the alginate/bioactive agent mixture obtained in step (a) to form chitosan/alginate nanoparticles comprising the bioactive agent that is associated via physicochemical interaction with a polymer,
wherein the chitosan/alginate nanoparticles are dispersed in the solution obtained in step (c).

According to a second aspect the invention provides the method according to the first aspect further comprising:
(d) mixing the formed chitosan/alginate nanoparticles comprising the bioactive agent that is associated via physicochemical interaction with a polymer that are dispersed in the solution obtained in step (c) with a second alginate solution to obtain a mixture of the formed chitosan/alginate nanoparticles; and
(e) adding the mixture of the formed chitosan/alginate nanoparticles obtained in step (d) into a second calcium chloride solution under mixing to obtain acid resistant alginate macrobeads containing the chitosan/alginate nanoparticles;
(f) optionally drying the acid resistant alginate macrobeads obtained in step (e).

According to a third aspect the invention provides a method according to the second aspect, wherein step (e) comprises mixing the mixture of the chitosan/alginate nanoparticles formed in step (d) with the second calcium chloride solution in an inline mixer.

According to a fourth aspect the invention provides the method of the second or third aspect, wherein chitosan is added to the second calcium chloride solution used in step (e) prior to step (e).

According to a fifth aspect the invention provides the method of the fourth aspect, wherein the second calcium chloride solution is prepared by mixing the second calcium chloride solution with chitosan under stirring.

According to a 6^{th} aspect, the invention provides the method of any of aspects 1 to 5, wherein the bioactive agent is an immunogen.

According to a 7^{th} aspect, the invention provides the method of aspect 6, wherein the immunogen is an immunogenic protein, an immunogenic peptide or a lipid antigen.

According to an 8^{th} aspect, the invention provides the method of aspect 7, wherein the immunogen is a membrane-associated protein or a lipid antigen.

According to a 9^{th} aspect, the invention provides the method of aspect 7 or 8, wherein the immunogen is a viral protein or a fragment thereof.

According to a 10^{th} aspect, the invention provides the method of aspect 8 or 9, wherein the membrane-associated protein is the spike protein of the Porcine Epidemic Diarrhea virus (PEDV) or a fragment thereof.

According to an 11^{th} aspect, the invention provides the method of aspect 1, wherein the polymer that is used for association of the bioactive agent via physicochemical interaction is a cationic polymer forming polyplexes with the bioactive agent.

According to a 12^{th} aspect, the invention provides the method of aspect 11, wherein prior to step (a) the method comprises mixing a solution of the bioactive agent in physiologically acceptable buffer with a solution of the cationic polymer in physiologically acceptable buffer to obtain a dispersion comprising a plurality of polyplexes.

According to a 13^{th} aspect, the invention provides the method of aspect 12, wherein the dispersion formed comprises as cationic polymer polyethyleneimine in a concentration of about 0.8 µg/ml to about 3000 µg/ml (w/v) of polyethyleneimine relative to the total volume of the dispersion that comprises the plurality of polyplexes.

According to a 14^{th} aspect, the invention provides the method of aspect 13, wherein the dispersion formed comprises polyethyleneimine in a concentration of about 40 µg/ml to about 1620 µg/ml (w/v) of polyethyleneimine relative to the total volume of the dispersion that comprises the plurality of polyplexes.

According to a 15^{th} aspect, the invention provides the method of aspect 13 or 14, wherein the dispersion formed comprises the bioactive agent in a concentration of about 6.25µg/ml to about 200 µg/ml (w/v) of the bioactive agent relative to the total volume of the dispersion that comprises the plurality of polyplexes.

According to a 16^{th} aspect, the invention provides the method of aspect 15, wherein the dispersion formed comprises the bioactive agent in a concentration of about 12.5 µg/ml to about 50 µg/ml (w/v) of the bioactive agent relative to the total volume of the dispersion that comprises the plurality of polyplexes.

According to a 17^{th} aspect, the invention provides the method of aspect 13 to 16, wherein the cationic polymer comprises polyethyleneimine with the molecular weight 5 to 250kDa, more preferably 5 to 25kDa.

According to an 18^{th} aspect, the invention provides the method of aspect 11 to 17, wherein the bioactive agent comprises plasmid DNA, protein, antigen, particulate carrier system, virus or a combination thereof.

According to a 19^{th} aspect, the invention provides the method of aspect 18, wherein the plasmid DNA comprises PEDV spike gene.

According to a 20^{th} aspect, the invention provides the method of aspect 19, wherein the protein comprises PEDV spike protein.

According to a 21^{st} aspect, the invention provides the method of aspect 18, wherein the virus comprises live virus, genetically modified virus, or an attenuated virus particle.

According to a 22^{nd} aspect, the invention provides the method of aspect 18 wherein the virus comprises PED virus.

According to a 23^{rd} aspect, the invention provides the method of any of aspects 6 to 22, wherein the first calcium chloride/chitosan solution contains chitosan in a concentration of about 0.0225 g/l to about 2 g/l relative to the total volume of the first calcium chloride/chitosan solution.

According to a 24^{th} aspect, the invention provides the method of aspect 23, wherein the first calcium chloride/chitosan solution contains chitosan in a concentration of about 0.0225 g/l to about 1 g/l relative to the total volume of the first calcium chloride/chitosan solution.

According to a 25^{th} aspect, the invention provides the method of aspects 6 to 22, wherein the first alginate solution contains alginate in a concentration of about 1 to 100 µg/ml alginate relative to the total volume of the first alginate solution.

According to a 26^{th} aspect, the invention provides the method of aspect 25, wherein the first alginate solution contains alginate in a concentration of about 50 to 75 µg/ml alginate relative to the total volume of the first alginate solution

According to a 27^{th} aspect, the invention provides the method of any of aspects 6 to 26, wherein the first calcium chloride/chitosan solution contains calcium chloride in a concentration of about 0.28 g/l to about 3.2 g/l relative to the total volume of the first calcium chloride/chitosan solution.

According to a 28^{th} aspect, the invention provides the method of any of aspects 6 to 26, wherein the second alginate solution contains alginate in a concentration of about 5 g/l to about 120 g/l relative to the total volume of the second alginate solution.

According to a 29^{th} aspect, the invention provides the method of aspect 28, wherein the second alginate solution contains alginate in a concentration of about 30 g/l to about 50 g/l relative to the total volume of the second alginate solution.

According to a 30^{th} aspect, the invention provides the method of any of aspects 6 to 29, wherein the second calcium chloride solution contains calcium chloride in a concentration of at least about 0.0005 g/l relative to the total volume of the second calcium chloride solution.

According to a 31^{st} aspect, the invention provides the method of aspect 30, wherein the second calcium chloride solution contains calcium chloride in a concentration of about 7.5 g/l to about 20 g/l relative to the total volume of the second calcium chloride solution.

According to a 32^{nd} aspect, the invention provides the method of any of aspects 6 to 31, wherein the second calcium chloride solution contains chitosan in a concentration of about 0.0005 g/l to about 25 g/l relative to the total volume of the second calcium chloride solution.

According to a 33^{rd} aspect, the invention provides the method of aspect 32, wherein the second calcium chloride solution contains chitosan in a concentration of about 5 g/l to about 15 g/l relative to the total volume of the second calcium chloride solution.

According to a 34^{th} aspect, the invention provides a primer set for amplifying the PEDV spike gene comprising: GCCATGGCTGTTTATTCTGTTACG (SEQ ID NO: 1) and TTAGTGATGGTGATGGTGATGTGGGTTCTTACATGCTGCAGT (SEQ ID NO: 2).

According to a 35^{th} aspect, the invention provides a primer set for amplifying the PEDV spike gene comprising: CTGGGATCCGTGCTGTTTATTCTGTTACG (SEQ ID NO: 3) and CTAGTCGACTCAGTGATGGTGATGGTGATGTGGGTTCTTACATGCTGCAGTG (SEQ ID NO: 4).

According to a 36^{th} aspect, the invention provides a chitosan/alginate nanoparticle, the nanoparticle comprising a bioactive agent, wherein the bioactive agent is associated via physicochemical interaction with a polymer, wherein the nanoparticle is obtainable by the method as described in aspects 1 and 6 to 22.

According to a 27^{th} aspect, the invention provides a chitosan/alginate nanoparticle of aspect 36, the nanoparticle comprising a cationic polymer, wherein the cationic polymer forms polyplexes with the bioactive agent.

According to a 38^{th} aspect, the invention provides an acid resistant alginate macrobead comprising nanoparticles comprising bioactive agent, wherein the bioactive agent is associated via physicochemical interaction with a polymer, wherein the macrobead is obtainable by the method as described in aspects 1 to 33.

According to a 39^{th} aspect, the invention provides an acid resistant chitosan/alginate macrobead of aspect 38 comprising nanoparticles comprising a cationic polymer, wherein the cationic polymer forms polyplexes with the bioactive agent.

According to a 40^{th} aspect, the invention provides a chitosan/alginate nanoparticles as defined in any of aspects 36 to 37 or of an acid resistant alginate macrobead as defined in any of aspects 38 to 39 for use in eliciting an immune response in an animal.

According to a 41^{st} aspect, the invention provides the nanoparticle or macrobead for the use of aspect 40, wherein the animal is a mammal or a bird.

According to a 42^{nd} aspect, the invention provides the nanoparticle or macrobead for the use of aspect 41, wherein the animal is a mammal is a human or a farm animal.

According to a 43^{rd} aspect, the invention provides the nanoparticle or macrobead for the use of aspect 42, wherein the farm animal is selected from the group consisting of a pig, a cow, a sheep, a horse, a dog, and a cat.

According to a 44^{th} aspect, the invention provides the nanoparticle or macrobead for the use of aspect 41, wherein the bird is selected from the group consisting of a chicken, a duck, a goose, and a turkey.

### BRIEF DESCRIPTION OF THE DRAWINGS

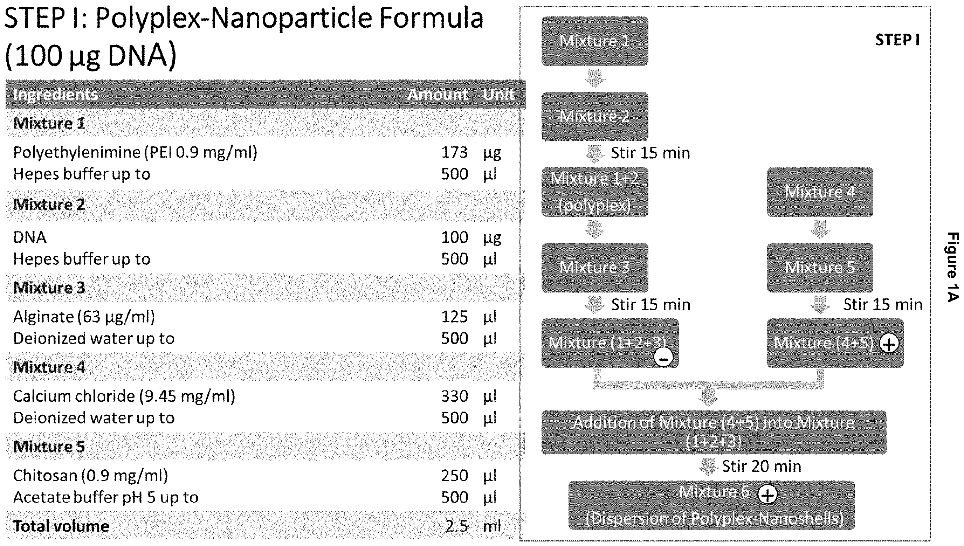

**Fig. 1A** shows a flowchart of the nanoparticle production using polyplexes as well as an example for the composition of the different solutions. For ease of the reader, the different mixtures are compared with the nomenclature used within this description: "Mixture 1" is the "solution of a cationic polymer", "mixture 2" is the "physiologically acceptable buffer including a bioactive compound (not shown)", "mixture 3" is the "first alginate solution", "mixture 4" is the "first calcium chloride solution", "mixture 5" is the "first chitosan solution" and "mixture 6" are the "chitosan/alginate nanoparticles comprising the bioactive agent that is associated via physicochemical interaction with a polymer, wherein the chitosan/alginate nanoparticles are dispersed in the solution".

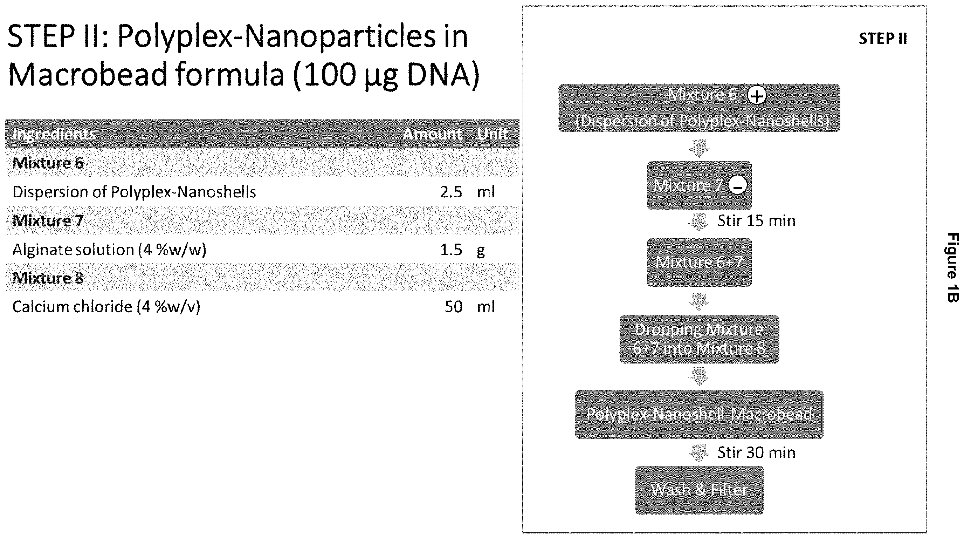

**Fig. 1B** shows a flowchart of the macrobead production using polyplexes as well as an example for the composition of different solutions. For ease of the reader, the different mixtures are compared with the nomenclature used within this description: "mixture 6" is the result of Fig. 1A and the "chitosan/alginate nanoparticles comprising the bioactive agent that is associated via physicochemical interaction with a polymer, wherein the chitosan/alginate nanoparticles are dispersed in the solution". "Mixture 7" is the "second alginate solution" and "mixture 8" the "second calcium chloride solution".
**Fig. 2A** illustrates the results of a gene transfection using polyplexes and nanoparticles containing polyplexes viewed by a fluorescence microscope.
**Fig. 2B** illustrates the results of a gene transfection using polyplexes and nanoparticles containing polyplexes measured by FACS.
**Fig. 2C** illustrates the results of a gene transfection using polyplexes and nanoparticles containing polyplexes by comparing the intensity of GFP fluorescence.
**Fig. 3** illustrates percentage of cell viability with polyplexes and other nanoparticles containing polyplexes using 4 different formulations.
**Fig. 4A** illustrates the effect of different chitosan concentrations on BHK cell transfection at concentrations of 4.5, 9, 18% chitosan depicted in GFP fluorescence measured by FACS.
**Fig. 4B** illustrates the effect of different chitosan concentrations on BHK cell transfection at concentrations of 4.5, 9, 18% chitosan depicted as relative fluorescence intensity.
**Fig. 5** illustrates the integrity of DNA in macrobeads in simulated gastric fluid pH 1.2 at time points 0.5, 1, 1.5, 2, 4, 6 hours.
**Fig. 6****:** illustrates the integrity of DNA in macrobead in simulated intestinal fluid pH 7.4 at time points 0.5, 1, 1.5, 2, 4, 6 hours.

### DETAILED DESCRIPTION OF THE INVENTION

The solution of the present invention is described in the following, exemplified in the appended examples, illustrated in the Figures and reflected in the claims.

The present invention provides a method for producing acid resistant macrobeads containing nanoparticles, wherein the nanoparticles comprise a bioactive agent, wherein the bioactive agent is associated via physicochemical interaction with a polymer. Further, the invention relates to a primer set for amplifying the PEDV spike gene, chitosan/alginate nanoparticles, acid-resistant alginate macrobeads and a PEDV antigen. In addition, the invention provides the use of a PEDV antigen for producing a chitosan/alginate nanoparticle or acid resistant macrobead.

An acid resistant macrobead containing nanoparticles may have the ability to deliver any types of antigen to immune cells including Microfold (M) cells that are found in the follicle-associated epithelium of the payer's patch of the small and large intestines. M cells are highly specialized cells playing a central role in the induction of mucosal immune response. The acid resistant macrobead containing nanoparticles may prevent the degradation of a bioactive agent, more specifically vaccine-related antigen, by providing double protection. The bioactive agent is encapsulated by the nanoparticles and the nanoparticles may be further encapsulated by the acid-resistant macrobead. The delivery of bioactive agent is increased, because the acid resistant macrobead containing nanoparticles protects the bioactive agent from being degraded in the acidic environment of the gastrointestinal tract. Moreover, the nanoparticles possess a mucoadhesive property which increase the retention time of the nanoparticles and subsequently increase the uptake by the immune cells such as M cells. Once the bioactive agent is delivered to M cells, the bioactive agent induces a mucosal immune response.

First, we would like to outline the principle of macrobead production. In a first step, the nanoparticles are produced. They comprise the bioactive reagent, which is associated with a polymer. This bioactive reagent associated with a polymer is encapsulated in a shell made of alginate and chitosan forming the nanoparticles. The size of the nanoparticles can be controlled by various parameters like stirring, temperature or concentration of alginate or the bioactive reagent. In a second step the nanoparticles are encapsulated in a second shell made of alginate forming the macrobeads. Fig. 1 shows a flowchart of the production of macrobeads starting from polyplexes.

The nanoparticles of the invention are made by mixing a solution comprising the bioactive reagent that is associated via physicochemical interaction with a polymer with a first alginate solution to obtain an alginate/bioactive agent mixture. Another mixture, the first calcium chloride/chitosan is also prepared. In a final step of nanoparticle production the first calcium chloride/chitosan solution is added to the alginate/bioactive agent mixture or the alginate/bioactive agent mixture is added to the first calcium chloride/chitosan solution. The resulting chitosan/alginate nanoparticles are dispersed in the solution obtained in the last step (see e.g. Fig. 1A). The nanoparticles of the invention preferably have a diameter between 150 and 300 nm, more preferably a diameter between 180 and 250 nm or in another embodiment preferably 230 to 240 nm.

The term "nanoparticle" as used herein describes the inner compartment of a macrobead. It is a first packaging of the bioactive agent associated via physicochemical interaction with a polymer. The packaging is made by alginate and chitosan. Nanoparticles provide acid resistance and facilitate the transfer of the bioactive reagent into the gut and/or via the mucosa into the blood system.

Accordingly, the present invention relates to a method for producing acid resistant macrobeads containing nanoparticles, wherein the nanoparticles comprise a bioactive agent, wherein the bioactive agent is associated via physicochemical interaction with a polymer, the method comprising:
(a) mixing a solution comprising the bioactive agent that is associated via physicochemical interaction with a polymer with a first alginate solution to obtain an alginate/bioactive agent mixture
(b) mixing a first calcium chloride solution with a first chitosan solution to obtain a first calcium chloride/chitosan solution and
(c) adding the first calcium chloride/chitosan solution obtained in step (b) to the alginate/bioactive agent mixture obtained in step (a) to form chitosan/alginate nanoparticles comprising the bioactive agent that is associated via physicochemical interaction with a polymer,
wherein the chitosan/alginate nanoparticles are dispersed in the solution obtained in step (c).

The nanoparticles of the invention may be further encapsulated into a macrobead. The term "macrobead" comprises at least one nanoparticle that is encapsulated by a second alginate capsule or layer. This ensures resistance against an acidic environment like in the stomach, so that the bioactive agent can safely be transported to the intestines. Macrobeads may be produced by mixing the chitosan/alginate nanoparticles produced in the first place with a second alginate solution. This mixture is then added to a second calcium chloride solution under mixing to obtain acid resistant alginate macrobeads containing the chitosan/alginate nanoparticles (see e.g. Fig. 1 B). Optionally, chitosan may be added to the second calcium chloride solution to form chitosan/alginate macrobeads. If desired, the macrobeads may be dried.

Accordingly, the method of producing acid resistant macrobeads containing nanoparticles of the invention, may further comprise:
(d) mixing the formed chitosan/alginate nanoparticles comprising the bioactive agent that is associated via physicochemical interaction with a polymer that are dispersed in the solution obtained in step (c) with a second alginate solution to obtain a mixture of the formed chitosan/alginate nanoparticles; and
(e) adding the mixture of the formed chitosan/alginate nanoparticles obtained in step (d) into a second calcium chloride solution under mixing to obtain acid resistant alginate macrobeads containing the chitosan/alginate nanoparticles;
(f) optionally drying the acid resistant alginate macrobeads obtained in step (e).

"Mixing" as used herein may also refer to stirring. Further, the addition of the mixture of the formed chitosan/alginate nanoparticles into a second calcium chloride solution may comprise in one embodiment mixing the mixture of the chitosan/alginate nanoparticles and the second alginate solution with the second calcium chloride solution in an inline mixer. In an alternative embodiment, the addition of the mixture of the formed chitosan/alginate nanoparticles into a second calcium chloride solution may comprise dripping mixture of the chitosan/alginate nanoparticles into the second calcium chloride solution, optionally under stirring. Stirring may control the size of the formed macrobeads and may also provide for homogeneity in size and shape of the macrobeads. Macrobeads of the invention preferably have a diameter of 0.5 to 10 mm, more preferably a diameter of 1 to 5 mm and most preferably a diameter of 2 to 3 mm.

In one embodiment, chitosan is added to the second calcium chloride solution. In this embodiment, the second calcium chloride solution is preferably prepared by mixing the calcium chloride solution with chitosan under stirring.

The term "physicochemical interaction" as used herein describes the sum of intermolecular interactions between at least two different compounds. These intermolecular interactions include electrostatic interactions and/or van der Waals forces. Depending on the polarity of the involved compounds the ratio of these interactions might be different. In one embodiment of the invention, physicochemical interaction might be seen as an interaction between negatively charged DNA and a positively charged polymer.

The "first alginate solution" as used herein describes an aqueous solution comprising alginate. Alginate, the salt of alginic acid, is an anionic polysaccharide distributed widely in the cell walls of brown algae. Alginic acid is a linear copolymer with homopolymeric blocks of (1-4)-linked β-D-mannuronate (M) and its C-5 epimer α-L-guluronate (G) residues, respectively, covalently linked together in different sequences or blocks. The monomers can appear in homopolymeric blocks of consecutive G-residues (G-blocks), consecutive M-residues (M-blocks) or alternating M and G-residues (MG-blocks). The first alginate solution defines the nanoparticle. Preferably, the first alginate solution has an alginate concentration of 1 to 100 µg/ml alginate relative to the total volume of the first alginate solution, more preferably a concentration of about 50 to 75 µg/ml alginate relative to the total volume of the first alginate solution. When being brought into contact with calcium ions, the alginate starts to polymerize and form a gel-like structure. The "second alginate solution" as used herein also comprises alginate as defined in this paragraph but is not necessarily identical to the first alginate solution. The second alginate solution may have an alginate concentration of about 5 g/l to about 120 g/l or of about 30 g/l to about 50 g/l relative to the total volume of the second alginate solution.

The "first calcium chloride/chitosan solution" as used herein is an aqueous solution comprising calcium chloride and chitosan. The calcium is used to induce the polymerization of alginate. Chitosan is a mucoadhesive and increases the attachment of nanoparticles and macrobeads to the mucosa of the intestine. The first calcium chloride/chitosan solution may comprise chitosan in a concentration of about 0.0225 g/l to about 2 g/l or 0.0225 g/l to 1 g/l relative to the total volume of the first calcium chloride/chitosan solution. The first calcium chloride/chitosan solution preferably contains calcium chloride in a concentration of about 0.28 g/l to about 3.2 g/l relative to the total volume of the first calcium chloride/chitosan solution. The "second calcium chloride" solution as used herein describes a calcium chloride solution similar to the first calcium chloride/chitosan solution, but does not contain chitosan. Optionally, the second calcium chloride solution may also comprise chitosan. The second calcium chloride solution preferably contains calcium chloride in a concentration of at least about 0.0005 g/l or of about 7.5 g/l to about 80 g/l, more preferably of about 30 g/l to about 50 g/l, relative to the total volume of the second calcium chloride solution. Optionally, the second calcium chloride/chitosan solution contains chitosan in a concentration of about 0.0005 g/l to about 25 g/l or of about 5 g/l to about 15 g/l relative to the total volume of the second calcium chloride solution.

The macrobeads of the invention are especially useful for the transport of immunogens, e.g. to transport immunogens into the gut for use as a vaccine. In one embodiment, the "bioactive agent" is an immunogen. An immunogen is a protein or substance that induces a response of the immune system specifically directed against this specific immunogen. Such an immunogen might be any protein or chemical compound. In a more preferred embodiment, the immunogen is an immunogenic protein, an immunogenic peptide or a lipid antigen. Even more preferably, the immunogen is a membrane-associated protein or a lipid antigen and most preferably, the immunogen is a viral protein or a fragment thereof.

The immunogen may be derived from a viral or bacterial pathogen. Examples for viral pathogens include, but are not limited to, hepatitis A virus, hepatitis B virus, hepatitis E virus, human papillomavirus, influenza virus, Japanese encephalitis virus, measles virus, mumps virus, polio virus, rabies virus, rotavirus, rubella virus, tick-borne encephalitis virus, varicella zoster virus, variola virus or yellow fever virus. Examples for bacterial pathogens include, but are not limited to, *Bacillus anthracis*, *Bordetella pertussis*, *Clostridium tetani*, *Corynebacterium diptheriae, Coxiella burnetii*, *Haemophilus influenzae, Mycobacterium tuberculosis*, *Neisseria meningitides, Salmonella typhi, Streptococcus pneumoniae,* or *Vibrio cholera.*

Porcine epidemic diarrhea virus (PEDV) is a coronavirus that infects the cells lining the small intestine of a pig causing porcine epidemic diarrhea, a condition of severe diarrhea and dehydration. Older hogs mostly get sick and lose weight after being infected, whereas newborn piglets usually die within five days of contracting the virus. As the mortality is very high for piglets, there is a need to provide a vaccine to eliminate PEDV in a population of pigs.

The immunogen of the invention might be any protein of the PEDV. In a preferred embodiment, the immunogen is the spike protein of PEDV or a fragment thereof. A preferred sequence of the spike protein of PEDV is deposited under UniProt accession number Q91AV1 (strain CV777). In its native state, this protein is split into two domains (S1 and S2). S1 is required for viral attachment to cells and S2 is needed for viral entry into the cells. A fragment of the spike protein of PEDV preferably is the S2 domain of the spike protein or at least a part of the S2 domain. More preferably, the fragment of the PEDV spike protein comprises amino acid residues 672-811 or amino acid residues 692-785 of the PEDV spike protein.

The polymer of the invention might be a cationic polymer forming polyplexes with the bioactive agent. Accordingly, the present invention relates to an embodiment of the invention, wherein the polymer that is used for association of the bioactive agent via physicochemical interaction is a cationic polymer forming polyplexes with the bioactive agent. The term "polyplex" as used herein describes the complex formed by the interaction of the cationic polymer and the bioactive agent, wherein the bioactive agent preferably is negatively charged to allow electrostatic, i.e. physicochemical, interactions. When using a cationic polymer as polymer in the method of the invention, then it is necessary to prepare the solution comprising the bioactive agent that is associated via physicochemical interaction with a polymer. Accordingly, the invention relates to the method of the invention, wherein prior to step (a) the method comprises mixing a solution of the bioactive agent in physiologically acceptable buffer with a solution of the cationic polymer in physiologically acceptable buffer to obtain a dispersion comprising a plurality of polyplexes (see Fig. 1A, Mixture 1 + 2 for the polyplex).

A "physiologically acceptable buffer" as used herein has a pH value similar to that of the body fluids, i.e. preferably has a pH value of about 7 to 7.8, more preferably of about 7.4. In addition, the physiologically acceptable buffer has an osmolarity and/or an osmolality similar to the body fluids like e.g. the blood, i.e. preferably an osmolarity and/or osmolality of 260 to 320 mOsm/kg or mOsm/l respectively, more preferably of about 290 mOsm/kg or 290 mOsm/l respectively.

The dispersion comprising a plurality of polyplexes may comprise different polymers at different concentrations. A preferred polymer is polyethyleneimine that is useful to complex negatively charged nucleic acids and to transfer nucleic acids across cell membranes. The dispersion comprising the plurality of polyplexes preferably comprises polyethyleneimine as a cationic polymer in a concentration of about 0.8 µg/ml to about 3000 µg/ml (w/v) of polyethyleneimine relative to the total volume of the dispersion that comprises the plurality of polyplexes, more preferably in a concentration of about 0.8 µg/ml to about 1620 µg/ml (see also Fig. 1A, mixture 6). In a further embodiment, the cationic polymer comprises polyethyleneimine with the molecular weight 5 to 250 kDa, preferably 5 to 25 kDa.

The dispersion comprising a plurality of polyplexes may also comprise the bioactive agent in different concentrations. Accordingly, the dispersion comprising a plurality of polyplexes preferably comprises the bioactive agent in a concentration of about 6.25 µg/ml to about 200 µg/ml (w/v) of the bioactive agent relative to the total volume of the dispersion that comprises the plurality of polyplexes, more preferably in a concentration of about 12.5 µg/ml to about 50 µg/ml.

The bioactive agent used within the invention may be plasmid DNA, a protein, an antigen, a particulate carrier system, virus or a combination thereof. Preferably, the plasmid DNA comprises PEDV spike gene. The protein preferably comprises PEDV spike protein. The virus preferably comprises live virus, genetically modified virus, or an attenuated virus particle, wherein the virus preferably comprises PED virus.

The invention also relates to a primer set for amplifying the PEDV spike gene comprising: GCCATGGCTGTTTATTCTGTTACG (SEQ ID NO: 1) and TTAGTGATGGTGATGGTGATGTGGGTTCTTACATGCTGCAGT (SEQ ID NO: 2). In another embodiment, the invention relates to a primer set for amplifying the PEDV spike gene comprising: CTGGGATCCGTGCTGTTTATTCTGTTACG (SEQ ID NO: 3) and CTAGTCGACTCAGTGATGGTGATGGTGATGTGGGTTCTTACATGCTGCAGTG (SEQ ID NO: 4). The product of this amplification may then be incorporated into nanoparticles.

The present invention also relates to a chitosan/alginate nanoparticle, the nanoparticle comprising a bioactive agent, wherein the bioactive agent is associated via physicochemical interaction with a polymer, wherein the nanoparticle is obtainable by a method of the invention. In a preferred embodiment, the nanoparticle comprises a cationic polymer, wherein the cationic polymer forms polyplexes with the bioactive agent.

The present invention also relates to an acid resistant alginate macrobead comprising nanoparticles comprising a bioactive agent, wherein the bioactive agent is associated via physicochemical interaction with a polymer, wherein the macrobead is obtainable by the method of the invention. In a preferred embodiment, the alginate macrobead comprising nanoparticles comprises a cationic polymer, wherein the cationic polymer forms polyplexes with the bioactive agent.

The chitosan/alginate nanoparticles and/or the alginate macrobeads of the invention may be used to elicit an immune response in an animal, i.e. they may be used as a vaccine. Accordingly, the invention relates to a chitosan/alginate nanoparticles and/or a alginate macrobeads of the invention for use in eliciting an immune response in an animal. Preferably, the animal is a mammal or bird, more preferably the animal is a human or a farm animal, more preferably the farm animal is selected from the group consisting of pig, a cow, a sheep, a horse, a dog, and a cat. Preferably, the bird is selected from the group consisting of a chicken, a duck, a goose, and a turkey.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications cited throughout the text of this specification (including all patents, patent application, scientific publications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

A better understanding of the present invention and of its advantages will be had from the following examples, offered for illustrative purposes only. The examples are not intended to limit the scope of the present invention in any way.

### EXAMPLES

### Example 1: Plasmid DNA containing PEDV spike gene

Plasmid DNA is prepared for use as a bioactive agent as part of the oral delivery method. In an embodiment, the bioactive agent comprises plasmid DNA encoding PEDV spike gene. Plasmid DNA encoding PEDV spike gene is constructed by inserting PEDV spike gene into an expression vector using the primer amplifying the gene region covering the nucleotide positions 123 to 4137. PEDV spike gene inserted into expression vector is either a synthesized sequence or directly obtained from amplification of PEDV, either live particle, RNA or cDNA materials. PEDV is isolated from the intestine, tissue or other organ that has been infected and multiplied in cell culture system using cell line. After the incubation and harvesting the virus, the RNA is extracted. DNA template of PEDV spike gene is prepared by reverse transcriptase polymerase chain reaction (RT-PCR). The first step is complimentary DNA (cDNA) construction from RNA. The second step is to amplify PEDV spike gene from cDNA in step one. A primer set for amplifying the PEDV gene for the PEDV spike gene inserted recombinant plasmid DNA is S1-2 forward primer 5'-GCCATGGCTGTTTATTCTGTTACG-3' (SEQ ID NO. 1) and S1-2 reverse primer 5'-TTAGTGATGGTGATGGTGATGTGGGTTCTTACATGCTGCAGT-3' (SEQ ID NO. 2).

The PCR product, which includes the PEDV spike gene, is ligated into an expression DNA vector and the PEDV spike gene inserted recombinant plasmid DNA is transformed into an expression system. PEDV spike gene inserted recombinant plasmid DNA expression was evaluated by process of DNA transfection into mammalian cell and protein confirmation by Western blot.

### Example 2: Recombinant PEDV spike proteins

In another embodiment, recombinant PEDV spike protein is prepared for use as a bioactive agent as part of the oral delivery method. A recombinant protein of PEDV is constructed by inserting PEDV spike gene into an expression vector using the primer amplifying the gene region covering the nucleotide position 123 to 4137. PEDV isolation, multiplication and RNA extraction are performed the same as described in example 1. For PEDV spike gene template preparation, the first step to construct cDNA from RNA sample by using commercial kit is the same as example 1. The second step is to amplify PEDV spike gene from cDNA in step one. A primer set used for amplifying the PEDV gene for the PEDV spike recombinant protein are the following: S₁₋₂ forward primer 5'-CTGGGATCCGTGCTGTTTATTCTGT TACG-3' (SEQ ID NO.: 3) and S₁₋₂ Reverse primer 5'-CTAGTCGACTCAGTGATGGTGATGGTGATGTGGGTTCTTACATGCTGCAGTG-3' (SEQ ID NO.: 4).

The PCR product, which has the PEDV spike gene, is ligated into the cloning vector and used as template. PEDV spike gene was amplified by PCR the same as above. PCR product and the cloning vector for protein expression are cut with restriction enzyme, ligate the PCR product of spike gene and transform the ligated recombinant plasmid into expression system. Expression system containing recombinant plasmid vector is induced to protein production and protein is purified.

### Example 3: Preparation of nanoparticles using polyplexes

First, the polyplex is formed by ionic interaction between positive charges of the amine nitrogen group of the PEI and the negative charges of the phosphate group of plasmid DNA. Then, the polyplex is encapsulated in the nanoparticle wherein the nanoparticle is a dispersion prepared by the ionic gelation method.

The steps are as followed:
**1.** Dissolve 30 µg of linear or branch PEI having the molecular weight range of 5 to 25 kDa in 100 µL of HEPES buffered saline and stir for 10 minutes.
**2.** Add 10 µg of bioactive agent, such as PEDV spike gene inserted recombinant plasmid DNA or recombinant protein of PEDV, to 100 µL of HEPES buffered saline and stir for 10 minutes.
**3.** Mix the bioactive agent solution in step 2 and the PEI solution in step 1 and stir for 15 minutes to obtain polyplexes.
**4.** Dissolve 1.5 µg sodium alginate in 100 µL of deionized water. Stir for 10 minutes to obtain first sodium alginate solution.
**5.** Mix the polyplex dispersion in step 3 and the first sodium alginate solution in step 4. Stir for 10 minutes.
**6.** Dissolve 0.6 mg calcium chloride in 100 µL of deionized water. Stir for 19 minutes to obtain the first calcium chloride solution.
**7.** Dissolve 45 µg chitosan in 100 µL of sodium acetate buffer pH 5. Stir for 20 minutes to obtain the first chitosan solution.
**8.** Mix the first calcium chloride solution in step 6 and the first chitosan solution in step 7. Stir for 15 minutes.
**9.** Add the mixture of the first calcium chloride solution and the first chitosan solution in step 8 into the polyplex dispersion in step 5. Stir for 30 minutes to form a plurality of nanoparticles dispersion.

General formula for the nanoparticles:
- PEI 0.0005 to 0.04 percent by mass per volume, molecular weight range of 5 to 25 kDa
- Bioactive agent 0.0002 to 0.025 percent by mass per volume
- Sodium alginate 0.00025 to 0.85 percent by mass per volume, molecular weight range less than 250 kDa
- Chitosan 0.003 to 0.45 percent by mass per volume, molecular weight range less than 220 kDa
- Calcium chloride 0.03-1.25 percent by mass per volume
- Sodium chloride 0.087 percent by mass per volume
- HEPES buffer 2 percent by volume per volume
- Sodium acetate 0.5 percent by mass per volume
- Acetic acid 0.63 percent by volume per volume
- water up to 100 percent by volume

The following tests have been performed on the polyplex and/or the nanoparticle containing polyplex.

### Transfection assay:

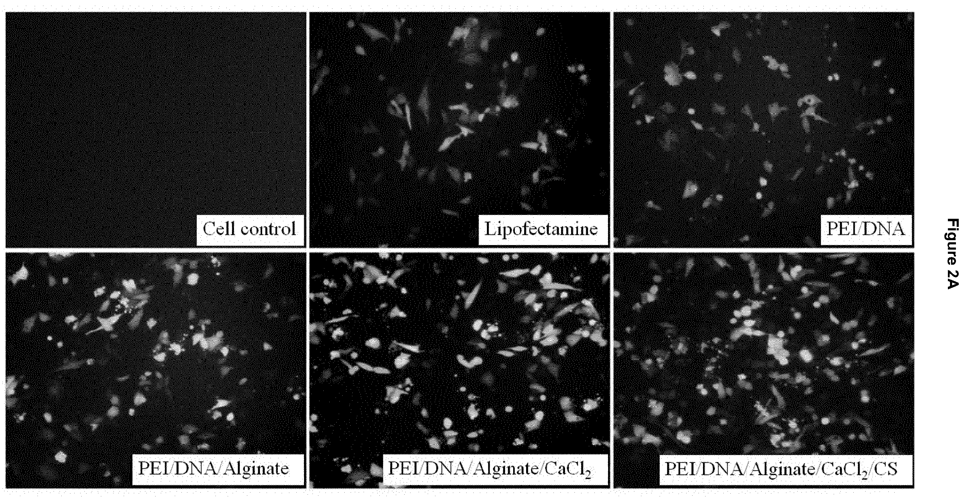

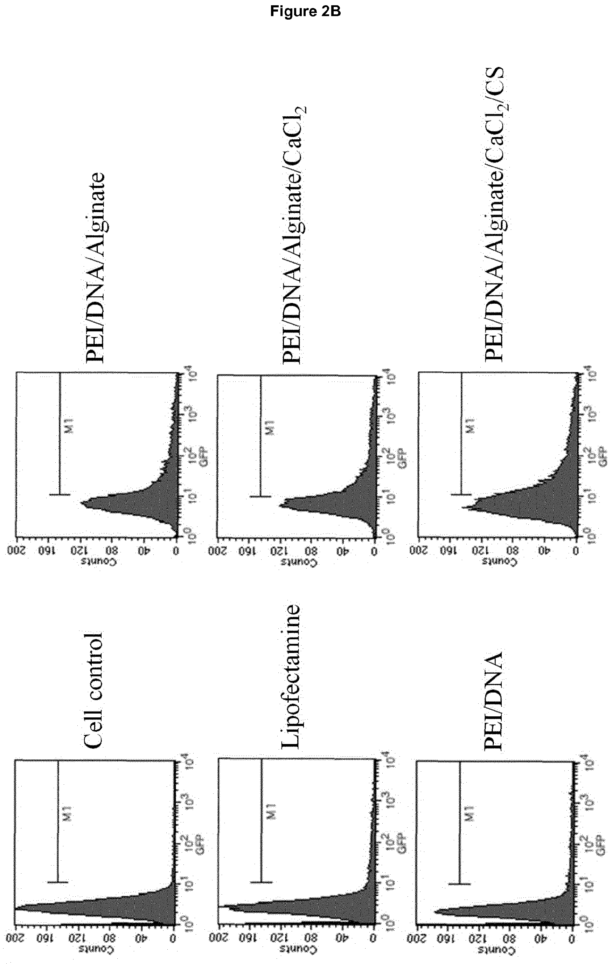

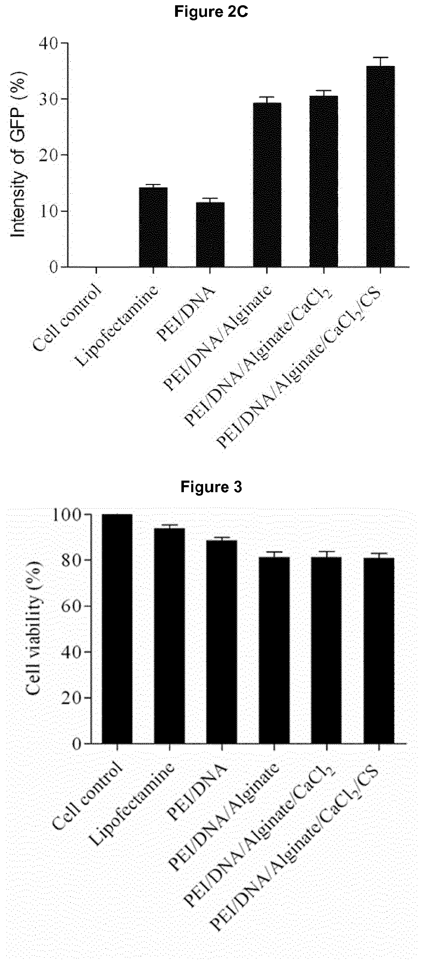

To determine the transfection efficiency, expression vector containing green fluorescent protein has been introduced into the BHK cell. Transfection efficiency is evaluated by fluorescent microscopy and flow cytometry. Gene transfection results of the polyplex showed that the concentration of PEI has an impact on the transfection efficiency: the higher the concentration the better the transfection. The PEI concentration of 2 % results in the highest GFP protein expression. Figure 2 shows gene transfection results of the polyplex and other nanoparticle containing polyplex, 1) PEI/DNA, 2) PEI/DNA/alginate, 3) PEI/DNA/calcium-alginate, and 4) PEI/DNA/calcium-alginate/chitosan (nanoparticle). Figure 2A shows the GFP protein expression in BHK cell under fluorescent microscope. Figure 2B is histograms of expressed GFP population by flow cytometry. Figure 2C demonstrates percentage of GFP intensity measured by flow cytometry. Lipofectamine is used as control. The results show that gene transfection of the formulation with alginate has higher efficiency than the formulation without alginate. The formulation of alginate, calcium and chitosan has the highest cell transfection efficiency.

### Cell viability assay:

Figure 3 demonstrates percentage of cell viability with polyplex and other nanoparticles containing polyplex comparing to non-treated cells and treated-lipofectamine cells. There are 4 formulations, 1) PEI/DNA, 2) PEI/DNA/Alginate, 3) PEI/DNA/Alginate/CaCl₂, and 4) PEI/DNA/Alginate/CaCl₂/CS. Non-treated cells are used as negative control and treated-lipofectamine cells are used as positive control. The results show that BHK cell viability treated with polyplex and others are greater than 80%.

### Effect of chitosan on BHK cell transfection:

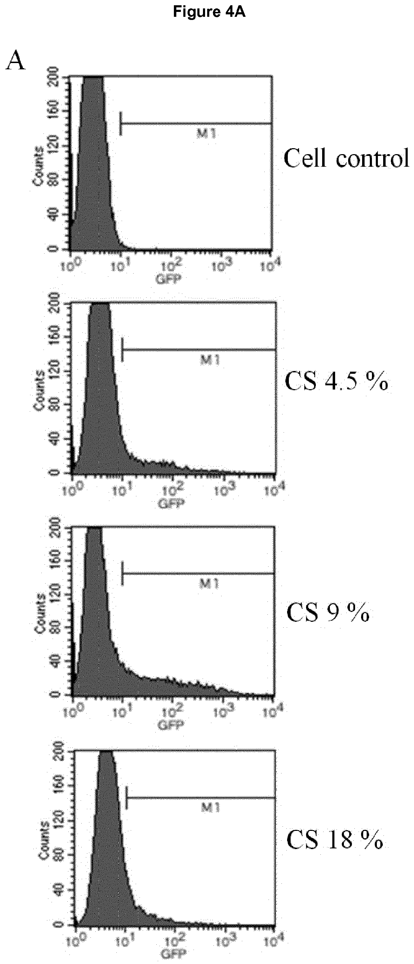

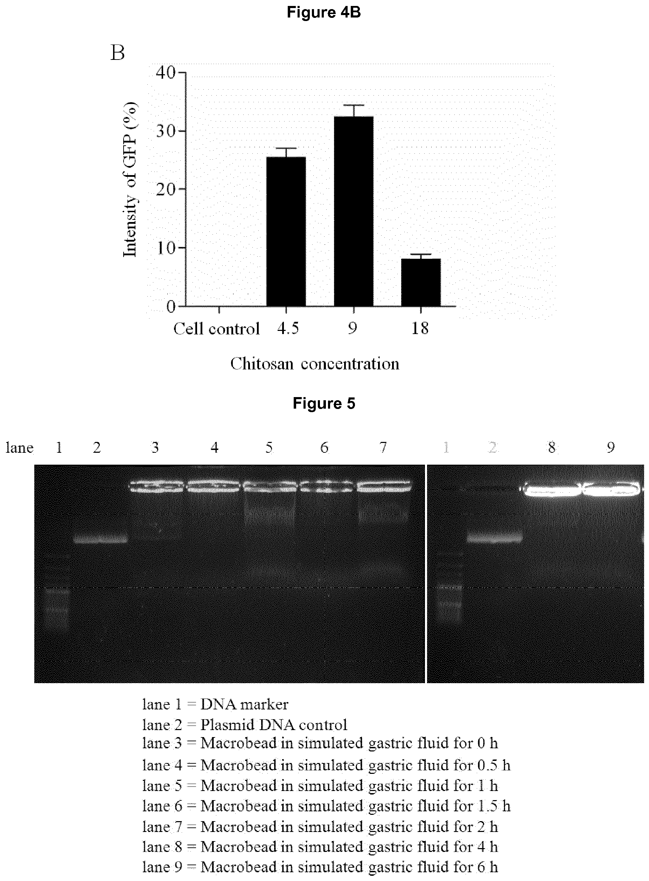

The effect of chitosan concentration on BHK cell transfection is shown in Figure 4. Figure 4A shows histogram of expressed GFP population by flow cytometry and Figure 4B shows percentage of GFP intensity measured by flow cytometry. At lower concentrations the transfection process is increased when the concentration of chitosan is increased. However, at higher concentrations, gene transfection does not increase as the amount of chitosan increases. The optimum chitosan concentration for gene transfection is 4.5 %.

### Example 4.1: Preparation of acid resistant macrobead containing nanoparticles

First, the nanoparticle dispersion is formed according to example 3. Then the following steps are performed to form the acid resistant macrobead. Disperse the nanoparticles dispersion into an alginate polymer solution to form a mixture of nanoparticles. Then, drop this mixture into a second calcium chloride solution under stirring. The acid resistant macrobeads are formed.

The steps are as follows:
1. Prepare the nanoparticle dispersion according to the example 3. Although the nanoparticle dispersion can be separated and washed in order to remove the excess calcium chloride and chitosan, which are the crosslinking agent for forming the macrobead, it is preferable to add nanoparticle dispersion directly into step number 2 below.
2. Dissolve 15 mg of sodium alginate in 500 µL of deionized water. Stir for 30 min to obtain the second sodium alginate solution.
3. Add the nanoparticle dispersion in step 1 into the second sodium alginate solution while stirring for 30 min to obtain the homogeneous dispersion of nanoparticle.
4. Dissolve 3 g of calcium chloride in 50 mL of deionized water. Stir for 19 min to obtain the second calcium chloride solution.
5. Dissolve 1 g of chitosan in 50 mL of sodium acetate buffer. Stir for 20 min to obtain the second chitosan solution.
6. Mix the second calcium chloride solution in step 4 and the second chitosan solution in step 5 Stir for 15 min.
7. Add the homogenous dispersion of nanoparticle in step 3 into the mixture of the second calcium chloride solution and the second chitosan solution in step 6. Stir for 30 min to obtain the suspension of acid resistant macrobead containing nanoparticles.
8. Filter the suspension of acid resistant macrobead containing nanoparticles through sieve and dry at 40 degree Celsius in the oven for 30 min.

General formula for the acid resistant macrobead containing the nanoparticles
- Nanoparticles dispersion (prepared as in example 3) 0.5 percent by volume per volume
- Sodium alginate 0.0002 to 12 percent by mass per volume, molecular weight range less than 250 kDa
- Chitosan 0.003 to 10 percent by mass per volume, molecular weight range less than 220 kDa
- Calcium chloride 0.03-20 percent by mass per volume
- Sodium acetate 0.5 percent by mass per volume
- Acetic acid 0.63 percent by volume per volume
- water up to 100 percent by volume

### Example 4.2: Preparation of acid resistant macrobead containing nanoparticles

First, the nanoparticle dispersion is formed according to example 3. Then the following steps are performed to form the acid resistant macrobeads that do not contain chitosan. Disperse the nanoparticles dispersion into an alginate polymer solution to form a mixture of nanoparticles. Then, drop this mixture into a second calcium chloride solution under stirring. The acid resistant macrobeads are formed. If not otherwise stated herein the used acids resistant macrobeads were produced according to example 4.2.

The steps are as follows:
1. Step 1 was performed according to step 1 of example 4.1.
2. Step 2 was performed according to step 2 of example 4.1.
3. Step 3 was performed according to step 1 of example 4.1.
4. Step 4 was performed according to step 4 of example 4.1.
5. Dissolve 3 g of calcium chloride in 100 mL of deionized water. Stir for 19 min to obtain the second calcium chloride solution.
6. Add the homogenous dispersion of nanoparticle in step 3 into the mixture of the second calcium chloride solution in step 5. Stir for 30 min to obtain the suspension of acid resistant macrobead containing nanoparticles.
7. Filter the suspension of acid resistant macrobead containing nanoparticles through sieve and dry at 40 degree Celsius in the oven for 30 min.

### Example 5: Study of acid resistant macrobeads in simulated gastric fluid pH 1.2

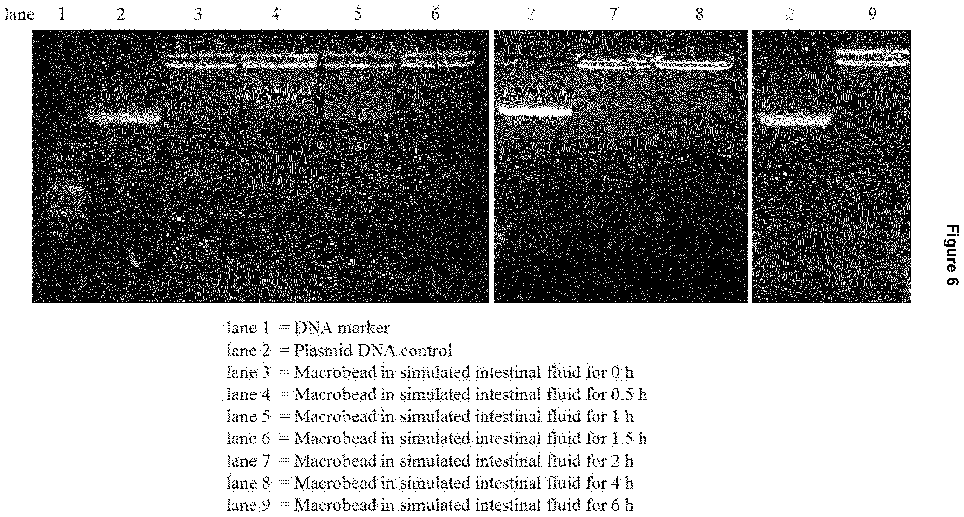

The stability study of plasmid DNA encapsulated in the macrobead is performed. The macrobead containing nanoparticles with plasmid DNA are incubated in simulated gastric fluid for 6 hours. Samples are removed at 0, 0.5, 1, 1.5, 2, 4, 6 hours and subjected to agarose gel electrophoresis. The results are shown in Figure 6. Lane 1 shows DNA marker. Lane 2 shows plasmid DNA control. Lane 3 shows macrobead containing nanoparticle incubated with simulated gastric fluid at various times.

The intensity of the band in well is used to investigate the effect of acidic condition on the acid resistant ability of the macrobead. There is no observation of the encapsulated DNA band until 6 hours. The result implies that macrobead can protect the encapsulated plasmid DNA from the acidic degradation for at least 6 hours as shown in Figure 5.

### Example 6: Study of acid resistant macrobeads in simulated intestinal fluid pH 7.4

A release study of plasmid DNA encapsulated in the macrobead is performed. The macrobead containing nanoparticles with plasmid DNA is incubated in simulated intestinal fluid for 6 hours. Samples are removed at 0, 0.5, 1, 1.5, 2, 4, 6 hours and subjected to agarose gel electrophoresis. The results are shown in Figure 6. Lane 1 shows DNA marker. Lane 2 shows plasmid DNA control. Lane 3 shows macrobead containing nanoparticles incubated with simulated intestinal fluid at various time.

The intensity of the band in well is used to investigate the effect of intestinal condition on the integrity of the macrobead. The result shows that the encapsulated plasmid DNA started to release from macrobead at 30 minutes and continued until 6 hours.

### Example 7: The efficacy of oral DNA and oral inactivated PEDV vaccines in the induction of mucosal immune response and protection against PEDV challenge

The acid resistant macrobead containing nanoparticles having plasmid DNA encoding spike gene and inactivated PEDV were used as oral vaccines to study the efficacy of these oral vaccines in the induction of mucosal immune response and protection against PEDV challenge. Fifty four pigs at approximately 3 weeks of age were procured from a herd free of PEDV. Upon arrival, all pigs were randomly allocated into 6 treatment groups of 9 pigs each, consisting of groups as following:
Group 1: Acid resistant macrobead containing nanoparticles having plasmid DNA encoding spike gene at low dosage (DNA-S12-L). Pigs in this group were administered orally twice at 0 and 14 days with acid resistant macrobead containing nanoparticles having plasmid DNA encoding spike gene at low dosage.
Group 2: Acid resistant macrobead containing nanoparticles having plasmid DNA encoding spike gene at high dosage (DNA-S12-H). Pigs in this group were administered orally twice at 0 and 14 days with acid resistant macrobead containing plasmid DNA encoding spike gene at high dosage.
Group 3: Acid resistant macrobead containing inactivated PEDV vaccine at low dosage (KV-L). Pigs in this group were administered orally twice at 0 and 14 days with acid resistant macrobead containing inactivated PEDV at dosage of 10²TCID₅₀/ml.
Group 4: Acid resistant macrobead containing inactivated PEDV vaccine at high dosage (KV-H). Pigs in this group were administered orally twice at 0 and 14 days with acid resistant macrobead containing inactivated PEDV at dosage of 10⁵TCID₅₀/ml.
Group 5: Acid resistant macrobead containing nanoparticles having plasmid control. Pigs in this group were administered orally twice at 0 and 14 days with acid resistant macrobead containing nanoparticles having plasmid DNA containing no gene insertion in nanoparticle.
Group 6: Negative control. Pigs in this group were left as negative control and were administered orally with mock media.

At 14 and 28 days post vaccination (DPV), 3 pigs in each group were euthanized. Mesenteric lymph node were collected and subjected for mononuclear cells (MNC) isolation. Mucosal immunity including IgA secreting cells and the percentages of IFN-gamma producing cells were determined using ELISPOT assay and intracellular flow cytometry, respectively.

At 28 DPV, all remaining 6 pigs in each group were divided into 2 sub-groups of 3 pigs each and orally challenged with 2 mL of PEDV, either classical or new PEDV variant, with the challenge dosage of 10⁵ TCID₅₀/ml. Following challenge, pigs were individually monitored for clinical diarrhea for 7 consecutive days on daily basis. Clinical diarrhea score from 1 to 5 as described following were given to each pig.
Score 1: healthy
Score 2: nausea, vomit and pasty feces
Score 3: semi-liquid without a formed consistency and emaciated
Score 4: watery content, lethargy, gaunt and mild-moderate dehydration
Score 5: watery content, lethargy, gaunt and severe dehydration

Rectal swab samples were collected at 5 and 7 days post challenge (DPC) and assayed for the presence of PEDV in fecal samples using polymerase chain reaction. Following a 7-day daily clinical observation, all pigs were euthanized.

The efficacy of the oral vaccination was evaluated by mucosal immune response. The results of the mucosal immune response as determined by the number of IFN- γ producing and Immunoglobulin A (IgA) secreting cells in mesenteric lymph nodes demonstrated that the acid resistant macrobead containing nanoparticles having plasmid DNA encoding spike gene and inactivated PEDV can induce mucosal immune response as evidenced by increased number of IFN- γ producing and IgA secreting cells in mesenteric lymph nodes at 28 DPV (Table 7). The number of IFN- γ producing cells in mesenteric lymph nodes at 28 DPV suggests that the response was dose-related and DNA-S12-H has highest response compared to the other groups as shown in Table 1. DNA-S12-L induces similar magnitude of response compared to KV-H. Interestingly, there is no IFN- γ producing cells observed in group KV-L, suggesting that the lower dosage of oral KV might not induce the mucosal response.

**Table 1: The percentage of IFN-γ secreting cells in mesenteric lymph nodes as measured by flow cytometry at 28 days post vaccination (DPV) and immunoglobulin A (IgA) secreting cells as measured by ELISPOT assay at 28 DPV.**

| **Groups** | **Treatments** | **IFN-γ (%) at 28 DPV** | **IgA secreting cells at 28 DPV (spots/10⁶ mononuclear cells)** |
|---|---|---|---|
| 1 | DNA-L | 2.44 | 14 |
| 2 | DNA-H | 9.96 | 46 |
| 3 | KV-L | Not Detected | 1 |
| 4 | KV-H | 4.98 | 5 |
| 5 | Plasmid control | Not Detected | 0 |
| 6 | Negative | Not Detected | 0 |

Pigs orally administered with the acid resistant macrobead containing nanoparticles having plasmid DNA encoding spike gene or inactivated PEDV were protected against oral PEDV challenge. The results of the clinical diarrhea score demonstrated that pigs in oral vaccination groups displayed no diarrhea (Table 2). Oral DNA vaccination, low and high dosage display no fecal shedding against classical or new variant. In the oral inactivated vaccine groups, only high dosage group displays no fecal shedding in the classical variant only.

**Table 2: The summary of clinical diarrhea score (CS) following challenge with either classical or new PEDV variant and the presence of PEDV in fecal samples as determined RT-PCR by 5 and 7 days post challenge (DPC).**

| Groups | Treatment groups | Classical PEDV variant | | | New PEDV variant | | |
|---|---|---|---|---|---|---|---|
| | | Clinical diarrhea score | Fecal shedding at 5 DPC | Fecal shedding at 7 DPC | Clinical diarrhea score | Fecal shedding at 5 DPC | Fecal shedding at 7 DPC |
| 1 | DNA-L | 1 | No | No | 1 | No | No |
| 2 | DNA-H | 1 | No | No | 1 | No | No |
| 3 | KV-L | 1 | No | No | 1 | Yes | No |
| 4 | KV-H | 1 | No | No | 1 | No | No |
| 5 | Plasmid control | 3 | Yes | No | 3 | Yes | No |
| 6 | Negative | 3 | Yes | No | 3 | Yes | No |

### Example 8: The efficacy of oral DNA, DNA+, inactivated, modified live and recombinant protein vaccines in the induction of mucosal immune response and protection against PEDV challenge

In the study, the efficacy of five oral vaccine preparations was evaluated. The comparison was also made with vaccines that are administered orally with and without nanoparticles. Five oral vaccine preparations included the acid resistant macrobead containing nanoparticles having plasmid DNA encoding spike gene, acid resistant macrobead containing nanoparticles having plasmid DNA encoding spike gene and recombinant spike protein attached on nanoparticles, inactivated PEDV, attenuated PEDV or recombinant spike protein. Five oral vaccine preparations were used as oral vaccines to study the efficacy of these oral vaccines in the induction of mucosal immune response and protection against PEDV challenge.

Seventy two pigs at approximately 3 weeks of age were procured from a herd free of PEDV. Upon arrival, all pigs were randomly allocated into 8 treatment groups of 9 pigs each. All 8 treatment groups included as following;
Group 1: PED DNA/Nanoparticle/Fresh macrobead. Group 1 was acid resistant macrobead containing nanoparticles having plasmid DNA encoding spike gene. Pigs in this group were administered orally twice at 0 and 14 days with acid resistant macrobead containing nanoparticles having plasmid DNA encoding spike gene.
Group 2: PED DNA/Nanoparticle plus S-protein/Fresh macrobead. Group 2 was acid resistant macrobead containing nanoparticles having plasmid DNA encoding spike gene and recombinant spike protein attached on nanoparticles. Pigs in this group were administered orally twice at 0 and 14 days with acid resistant macrobead containing nanoparticles having plasmid DNA encoding spike gene and recombinant protein attached on nanoparticles.
Group 3: Nanoparticle plus S-protein/Fresh macrobead. Group 3 was acid resistant macrobead containing nanoparticles having recombinant spike protein attached on nanoparticles. Pigs in this group were administered orally twice at 0 and 14 days with acid resistant macrobead containing nanoparticles having recombinant spike protein attached on nanoparticles.
Group 4: Inactivated PEDV/Nanoparticle/ Fresh macrobead. Group 4 was acid resistant macrobead containing nanoparticles having inactivated PEDV. Pigs in this group were administered orally twice at 0 and 14 days with acid resistant macrobead containing nanoparticles having inactivated PEDV.
Group 5: Inactivated PEDV/ Fresh macrobead. Group 5 was acid resistant macrobead containing inactivated PEDV. Pigs in this group were administered orally twice at 0 and 14 days with acid resistant macrobead containing nanoparticles having inactivated PEDV.
Group 6: Attenuated PEDV/Nanoparticle/ Fresh macrobead. Group 6 was acid resistant macrobead containing nanoparticles having attenuated PEDV. Pigs in this group were administered orally twice at 0 and 14 days with acid resistant macrobead containing nanoparticles having attenuated PEDV.
Group 7: Attenuated PEDV/ Fresh macrobead. Group 7 was acid resistant macrobead containing attenuated PEDV. Pigs in this group were administered orally twice at 0 and 14 days with acid resistant macrobead containing nanoparticles having attenuated PEDV.
Group 8: S-protein inside Nanoparticle /Fresh macrobead. Group 8 was acid resistant macrobead containing nanoparticles having recombinant spike protein. Pigs in this group were administered orally twice at 0 and 14 days with acid resistant macrobead containing nanoparticles having recombinant spike protein.
Group 9: Negative control. Pigs in this group were left as negative control and were administered orally with mock media.

At 28 days post vaccination (DPV), 3 pigs in each group were euthanized. Mesenteric lymph node were collected and subjected for mononuclear cells (MNC) isolation. Mucosal immunity including IgA secreting cells and the percentages of IFN-gamma producing cells were determined using ELISPOT assay and intracellular flow cytometry, respectively.

At 28 DPV, all remaining 6 pigs in each group were divided into 2 sub-groups of 3 pigs each and orally challenged with 2 mL of PEDV, either classical or new PEDV variant, with the challenge dosage of 10⁵ TCID₅₀/ ml. Following challenge, pigs were individually monitored for clinical diarrhea for 7 consecutive days on daily basis. Clinical diarrhea score from 1 to 5 as described following were given to each pig.
Score 1: healthy
Score 2: nausea, vomit and pasty feces
Score 3: semi-liquid without a formed consistency and emaciated
Score 4: watery content, lethargy, gaunt and mild-moderate dehydration
Score 5: watery content, lethargy, gaunt and severe dehydration

Rectal swab samples were collected at 5 and 7 days post challenge (DPC) and assayed for the presence of PEDV in fecal samples using polymerase chain reaction. Following a 7-day daily clinical observation, all pigs were euthanized.

**Table 3: The percentage of IFN-γ secreting cells in mesenteric lymph nodes as measured by flow cytometry at 28 days post vaccination (DPV) and immunoglobulin (Ig)-A (IgA) secreting cells as measured by ELISPOT assay at 28 DPV.**

| **Groups** | **TreatmentsIFN-γ(%) at 28 DPV** | **IFN-γ(%) at 28 DPV** | **IgA secreting cells at 28 DPV (sport/10⁶ mononuclear cells)** |
|---|---|---|---|
| 1 | Plasmid DNA-PEDV/ Nanoparticles/ Fresh macrobead | 12.44 (0.027) | 44 |
| 2 | Plasmid DNA-PEDV/ Nanoparticle plus S-protein/ Nanoparticles/ Fresh macrobead | 9.96 (0.023) | 46 |
| 3 | Nanoparticle plus S-protein/ Fresh macrobead | 7.78 (0.003) | 23 |
| 4 | Inactivated PEDV/Nanoparticles/ Fresh macrobead | 4.98 (0.007) | 5 |
| 5 | Inactivated PEDV/ Fresh macrobead | Not Detected | 0 |
| 6 | Live PED virus/ Nanoparticle/ Fresh macrobead | 9.88 (0.017) | 29 |
| 7 | Live PED virus/ Fresh macrobead | 4.78 (0.033) | 12 |
| 8 | S-protein inside/ Nanoparticle / Fresh macrobead | 10.68 (0.020) | 13 |
| 9 | Negative control | Not Detected | 0 |

**Table 4: The summary of clinical diarrhea score (CS) following challenge with either classical or new PEDV variant and the presence of PEDV in fecal samples as determined RT-PCR by 5 and 7 days post challenge (DPC).**

| Group s | Treatment groups | Classical PEDV variant | | | New PEDV variant | | |
|---|---|---|---|---|---|---|---|
| | | Clinical diarrhe a score | Fecal sheddin g at 5 DPC | Fecal sheddin g at 7 DPC | Clinical diarrhe a score | Fecal sheddin g at 5 DPC | Fecal sheddin g at 7 DPC |
| 1 | Plasmid DNA-PEDV/ Nanoparticles/ Fresh macrobead | 1 | No | No | 1 | No | No |
| 2 | Plasmid DNA-PEDV/ Nanoparticle plus S-protein/ Nanoparticles/ Fresh macrobead | 1 | No | No | 1 | No | No |
| 3 | Nanoparticle plus S-protein/ Fresh macrobead | 1 | No | No | 1 | Yes | No |
| 4 | Inactivated PEDV/Nanoparticles / Fresh macrobead | 1 | No | No | 1 | No | No |
| 5 | Inactivated PEDV/ Fresh macrobead | 2 | Yes | No | 2 | Yes | No |
| 6 | Live PED virus/ Nanoparticle/ Fresh macrobead | 1 | No | No | 1 | No | No |
| 7 | Live PED virus/ Fresh macrobead | 2 | Yes | No | 2 | Yes | No |
| 8 | S-protein inside/ Nanoparticle / Fresh macrobead | 1 | No | No | 1 | No | No |
| 9 | Negative control | 4 | Yes | No | 3 | Yes | No |

### Example 9: Comparison of the efficacy in sows between oral DNA, oral inactivated and oral attenuated vaccines

A field efficacy study of the acid resistant macrobead containing nanoparticles having plasmid DNA encoding spike gene, inactivated PEDV or attenuated PEDV was performed in comparison to feedback, a common management practice that has been implemented in farms to control PED. PEDV infected intestine is collected from PEDV infected pigs and given orally to sows at pre-farrow at 12 to 13 weeks of gestation as Feedback. A comparison is also made with vaccine that is administered orally without macrobead and nanoparticle. Thirty are separated based on the stratification of parity into 6 groups of 5 sows. The study is performed in duplicate. The control is the Feedback method. The 6 treatment groups are as follows:
Group 1: Negative control. Pigs in this group have not been treated.
Group 2: Feedback. Pigs in this group are orally feedback twice at 12 and 14 weeks of gestation. Each feedback is administered once a day for 2 days.
Group 3: Acid resistant macrobead containing attenuated virus. Pigs in this group are vaccinated orally twice at 12 and 14 weeks of gestation with macrobead containing attenuated virus at the dosage of 10⁴ TCID₅₀/mL, for 5 ml. Each dose is administered once a day for 2 days.
Group 4: Attenuated virus in water. Pigs in this group are vaccinated orally twice at 12 and 14 weeks of gestation with attenuated virus in water at the dosage of 10⁴ TCID₅₀/mL, for 5 ml. Each dose is administered once a day for 2 days.
Group 5: Acid resistant macrobead containing inactivated virus. Pigs in this group are vaccinated orally twice at 12 and 14 weeks of gestation with macrobead containing killed virus at the dosage of 10⁵ TCID₅₀/mL, for 5 ml. Each dose is administered once a day for 2 days.
Group 6: Acid resistant macrobead containing nanoparticle having plasmid DNA encoding spike gene/High. Pigs in this group are vaccinated orally twice at 12 and 14 weeks of gestation with acid resistant macrobead containing nanoparticle having plasmid DNA encoding spike gene at high dosage. Each dose is administered once a day for 2 days.

The antibody responses in sows following oral vaccination compared to feedback. Antibody response in colostrum and milk samples collected between 3 hours and 7 days post-farrowing period are measured by ELISA IgG and IgA. The plate is coated with spike protein. Antibody response is also measure by viral neutralization assay.

As shown in Table 5 feedback induces a high response as demonstrated by viral neutralization titer and ELISA IgA, but the result is inconsistent. This can be due to varying dosage of antigen in feedback. Acid resistant macrobead containing attenuated virus and acid resistant macrobead containing nanoparticle having plasmid DNA/High induced the best response. Acid resistant macrobead containing killed virus and attenuated virus in water induces the second best response. Acid resistant macrobead containing killed virus and attenuated virus in water have comparable results. This can be due to the differences in the means of administration. Since the attenuated viruses in water are not covered they can be destroyed by the acidic environment in the stomach.

**Table 5 shows the results for the antibody responses in sows following oral vaccination compared to feedback. Antibody response in colostrum and milk samples collected between 3 hours and 7 days post-farrowing period are measured by ELISA IgG and IgA.**

| Groups | Replicate | IgG | | IgA | | Viral neutralization titer | |
|---|---|---|---|---|---|---|---|
| | | Colostrum | Milk | Colostrum | Milk | Colostrum | Milk |
| Negative control | 1 | 0.711 | 0.147 | 0.499 | 0.313 | 16 | 4 |
| | 2 | 2.044 | 0.471 | 0.362 | 0.366 | 32 | 16 |
| Feedback | 1 | 1.039 | 0.415 | 0.450 | 1.017 | 16 | 64 |
| | 2 | 0.418 | 0.121 | 0.338 | 0.207 | 16 | 16 |
| Acid resistant macrobead containing attenuated virus | 1 | 0.810 | 0.266 | 0.404 | 0.228 | 16 | 32 |
| | 2 | 0.880 | 0.630 | 0.334 | 0.488 | 64 | 16 |
| Attenuated virus in water | 1 | 2.072 | 1.334 | 1.279 | 0.891 | 16 | 32 |
| | 2 | 0.511 | 0.125 | 0.324 | 0.089 | 16 | 8 |
| Acid resistant macrobead containing inactivated virus | 1 | 1.814 | 0.384 | 0.632 | 0.150 | 64 | 16 |
| | 2 | 1.127 | 0.158 | 1.112 | 0.425 | 16 | 16 |
| Acid resistant macrobead containing nanoparticle having plasmid DNA-spike/High | 1 | 1.139 | 0.163 | 0.755 | 0.403 | 16 | 16 |
| | 2 | 1.698 | 0.158 | 0.171 | 0.280 | 32 | 16 |

The invention is also characterized by the following items:
1. A method for producing acid resistant macrobeads containing nanoparticles, wherein the nanoparticles comprise a bioactive agent, wherein the bioactive agent is associated via physicochemical interaction with a polymer, the method comprising:
   (a) mixing a solution comprising the bioactive agent that is associated via physicochemical interaction with a polymer with a first alginate solution to obtain an alginate/bioactive agent mixture
   (b) mixing a first calcium chloride solution with a first chitosan solution to obtain a first calcium chloride/chitosan solution and
   (c) adding the first calcium chloride/chitosan solution obtained in step (b) to the alginate/bioactive agent mixture obtained in step (a) to form chitosan/alginate nanoparticles comprising the bioactive agent that is associated via physicochemical interaction with a polymer,
   wherein the chitosan/alginate nanoparticles are dispersed in the solution obtained in step (c).
2. The method according to item 1 further comprising:
   (d) mixing the formed chitosan/alginate nanoparticles comprising the bioactive agent that is associated via physicochemical interaction with a polymer that are dispersed in the solution obtained in step (c) with a second alginate solution to obtain a mixture of the formed chitosan/alginate nanoparticles; and
   (e) adding the mixture of the formed chitosan/alginate nanoparticles obtained in step (d) into a second calcium chloride solution under mixing to obtain acid resistant alginate macrobeads containing the chitosan/alginate nanoparticles;
   (f) optionally drying the acid resistant alginate macrobeads obtained in step (e).
3. The method according to item 2, wherein step (e) comprises mixing the mixture of the chitosan/alginate nanoparticles formed in step (d) with the second calcium chloride solution in an inline mixer.
4. The method of item 2 or 3, wherein chitosan is added to the second calcium chloride solution used in step (e) prior to step (e).
5. The method of item 4, wherein the second calcium chloride solution is prepared by mixing the second calcium chloride solution with chitosan under stirring.
6. The method of any of items 1 to 5, wherein the bioactive agent is an immunogen.
7. The method of item 6, wherein the immunogen is an immunogenic protein, an immunogenic peptide or a lipid antigen.
8. The method of item 7, wherein the immunogen is a membrane-associated protein or a lipid antigen.
9. The method of item 7 or 8, wherein the immunogen is a viral protein or a fragment thereof.
10. The method of item 8 or 9, wherein the membrane-associated protein is the spike protein of the Porcine Epidemic Diarrhea virus (PEDV) or a fragment thereof.
11. The method of item 1, wherein the polymer that is used for association of the bioactive agent via physicochemical interaction is a cationic polymer forming polyplexes with the bioactive agent.
12. The method of item 11, wherein prior to step (a) the method comprises mixing a solution of the bioactive agent in physiologically acceptable buffer with a solution of the cationic polymer in physiologically acceptable buffer to obtain a dispersion comprising a plurality of polyplexes.
13. The method of item 12, wherein the dispersion formed comprises as cationic polymer polyethyleneimine in a concentration of about 0.8 µg/ml to about 3000 µg/ml (w/v) of polyethyleneimine relative to the total volume of the dispersion that comprises the plurality of polyplexes.
14. The method of item 13, wherein the dispersion formed comprises polyethyleneimine in a concentration of about 40 µg/ml to about 1620 µg/ml (w/v) of polyethyleneimine relative to the total volume of the dispersion that comprises the plurality of polyplexes.
15. The method of item 13 or 14, wherein the dispersion formed comprises the bioactive agent in a concentration of about 6.25µg/ml to about 200 µg/ml (w/v) of the bioactive agent relative to the total volume of the dispersion that comprises the plurality of polyplexes.
16. The method of item 15, wherein the dispersion formed comprises the bioactive agent in a concentration of about 12.5 µg/ml to about 50 µg/ml (w/v) of the bioactive agent relative to the total volume of the dispersion that comprises the plurality of polyplexes.
17. The method of item 13 to 16, wherein the cationic polymer comprises polyethyleneimine with the molecular weight 5 to 250kDa, more preferably 5 to 25kDa.
18. The method of item 11 to 17, wherein the bioactive agent comprises plasmid DNA, protein, antigen, particulate carrier system, virus or a combination thereof.
19. The method of item 18, wherein the plasmid DNA comprises PEDV spike gene.
20. The method of item 19, wherein the protein comprises PEDV spike protein.
21. The method of item 18, wherein the virus comprises live virus, genetically modified virus, or an attenuated virus particle.
22. The method of item 18 wherein the virus comprises PED virus.
23. The method of any of items 6 to 22, wherein the first calcium chloride/chitosan solution contains chitosan in a concentration of about 0.0225 g/l to about 2 g/l relative to the total volume of the first calcium chloride/chitosan solution.
24. The method of item 23, wherein the first calcium chloride/chitosan solution contains chitosan in a concentration of about 0.0225 g/l to about 1 g/l relative to the total volume of the first calcium chloride/chitosan solution.
25. The method of any of items 6 to 22, wherein the first alginate solution contains alginate in a concentration of about 1 to 100 µg/ml alginate relative to the total volume of the first alginate solution.
26. The method of item 25, wherein the first alginate solution contains alginate in a concentration of about 50 to 75 µg/ml alginate relative to the total volume of the first alginate solution.
27. The method of any of items 6 to 26, wherein the first calcium chloride/chitosan solution contains calcium chloride in a concentration of about 0.28 g/l to about 3.2 g/l relative to the total volume of the first calcium chloride/chitosan solution.
28. The method of any of items 6 to 27, wherein the second alginate solution contains alginate in a concentration of about 5 g/l to about 120 g/l relative to the total volume of the second alginate solution.
29. The method of item 28, wherein the second alginate solution contains alginate in a concentration of about 30 g/l to about 50 g/l relative to the total volume of the second alginate solution.
30. The method of any of items 6 to 29, wherein the second calcium chloride solution contains calcium chloride in a concentration of at least about 0.0005 g/l relative to the total volume of the second calcium chloride solution.
31. The method of item 30, wherein the second calcium chloride solution contains calcium chloride in a concentration of about 7.5 g/l to about 80 g/l, more preferably of about 30 g/l to about 50 g/l, relative to the total volume of the second calcium chloride/chitosan solution.
32. The method of any of items 6 to 31, wherein the second calcium chloride solution contains chitosan in a concentration of about 0.0005 g/l to about 25 g/l relative to the total volume of the second calcium chloride solution.
33. The method of item 32, wherein the second calcium chloride solution contains chitosan in a concentration of about 5 g/l to about 15 g/l relative to the total volume of the second calcium chloride/chitosan solution.
34. A primer set for amplifying the PEDV spike gene comprising: GCCATGGCTGTTTATTCTGTTACG (SEQ ID NO: 1) and TTAGTGATGGTGATGGTGATGTGGGTTCTTACATGCTGCAGT (SEQ ID NO: 2).
35. A primer set for amplifying the PEDV spike gene comprising: CTGGGATCCGTGCTGTTTATTCTGTTACG (SEQ ID NO: 3) and CTAGTCGACTCAGTGATGGTGATGGTGATGTGGGTTCTTACATGCTGCAGTG (SEQ ID NO: 4).
36. A chitosan/alginate nanoparticle, the nanoparticle comprising a bioactive agent, wherein the bioactive agent is associated via physicochemical interaction with a polymer, wherein the nanoparticle is obtainable by the method as described in items 1 and 6 to 22.
37. A chitosan/alginate nanoparticle of item 36, the nanoparticle comprising a cationic polymer, wherein the cationic polymer forms polyplexes with the bioactive agent.
38. An acid resistant alginate macrobead comprising nanoparticles comprising bioactive agent, wherein the bioactive agent is associated via physicochemical interaction with a polymer, wherein the macrobead is obtainable by the method as described in items 1 to 33.
39. An acid resistant alginate macrobead of item 38 comprising nanoparticles comprising a cationic polymer, wherein the cationic polymer forms polyplexes with the bioactive agent.
40. A chitosan/alginate nanoparticles as defined in any of items 36 to 37 or of an acid resistant alginate macrobead as defined in any of items 38 to 39 for use in eliciting an immune response in an animal.
41. The nanoparticle or macrobead for the use of item 40, wherein the animal is a mammal or a bird.
42. The nanoparticle or macrobead for the use of item 41, wherein the animal is a mammal is a human or a farm animal.
43. The nanoparticle or macrobead for the use of item 42, wherein the farm animal is selected from the group consisting of a pig, a cow, a sheep, a horse, a dog, and a cat.
44. The nanoparticle or macrobead for the use of item 41, wherein the bird is selected from the group consisting of a chicken, a duck, a goose, and a turkey.

## Claims

1. A method for producing acid resistant macrobeads containing nanoparticles, wherein the nanoparticles comprise a bioactive agent, wherein the bioactive agent is associated via physicochemical interaction with a polymer, the method comprising:
(a) mixing a solution comprising the bioactive agent that is associated via physicochemical interaction with a polymer with a first alginate solution to obtain an alginate/bioactive agent mixture
(b) mixing a first calcium chloride solution with a first chitosan solution to obtain a first calcium chloride/chitosan solution and
(c) adding the first calcium chloride/chitosan solution obtained in step (b) to the alginate/bioactive agent mixture obtained in step (a) to form chitosan/alginate nanoparticles comprising the bioactive agent that is associated via physicochemical interaction with a polymer,
wherein the chitosan/alginate nanoparticles are dispersed in the solution obtained in step (c).

2. The method according to claim 1 further comprising:
(d) mixing the formed chitosan/alginate nanoparticles comprising the bioactive agent that is associated via physicochemical interaction with a polymer that are dispersed in the solution obtained in step (c) with a second alginate solution to obtain a mixture of the formed chitosan/alginate nanoparticles; and
(e) adding the mixture of the formed chitosan/alginate nanoparticles obtained in step (d) into a second calcium chloride solution under mixing to obtain acid resistant alginate macrobeads containing the chitosan/alginate nanoparticles;
(f) optionally drying the acid resistant alginate macrobeads obtained in step (e).

3. The method according to claim 2, wherein step (e) comprises mixing the mixture of the chitosan/alginate nanoparticles formed in step (d) with the second calcium chloride solution in an inline mixer.

4. The method of claim 2 or 3, wherein chitosan is added to the second calcium chloride solution used in step (e) prior to step (e).

5. The method of any of claims 1 to 4, wherein the bioactive agent is an immunogen.

6. The method of claim 5, wherein the immunogen is an immunogenic protein, an immunogenic peptide or a lipid antigen.

7. The method of claim 6, wherein the immunogen is a membrane-associated protein or a lipid antigen, wherein the membrane-associated protein is preferably the spike protein of the Porcine Epidemic Diarrhea virus (PEDV) or a fragment thereof.

8. The method of claim 1, wherein the polymer that is used for association of the bioactive agent via physicochemical interaction is a cationic polymer forming polyplexes with the bioactive agent.

9. The method of claim 8, wherein prior to step (a) the method comprises mixing a solution of the bioactive agent in physiologically acceptable buffer with a solution of the cationic polymer in physiologically acceptable buffer to obtain a dispersion comprising a plurality of polyplexes.

10. The method of claim 9, wherein the bioactive agent comprises plasmid DNA, protein, antigen, particulate carrier system, virus or a combination thereof, wherein the plasmid DNA preferably comprises PEDV spike gene

11. A primer set for amplifying the PEDV spike gene comprising: GCCATGGCTGTTTATTCTGTTACG (SEQ ID NO: 1) and TTAGTGATGGTGATGGTGATGTGGGTTCTTACATGCTGCAGT (SEQ ID NO: 2).

12. A primer set for amplifying the PEDV spike gene comprising: CTGGGATCCGTGCTGTTTATTCTGTTACG (SEQ ID NO: 3) and CTAGTCGACTCAGTGATGGTGATGGTGATGTGGGTTCTTACATGCTGCAGTG (SEQ ID NO: 4).

13. A chitosan/alginate nanoparticle, the nanoparticle comprising a bioactive agent, wherein the bioactive agent is associated via physicochemical interaction with a polymer, wherein the nanoparticle is obtainable by the method as described in claims 1 and 5 to 7, wherein, preferably, when the nanoparticle comprises a cationic polymer, the cationic polymer forms polyplexes with the bioactive agent.

14. An acid resistant alginate macrobead comprising nanoparticles comprising bioactive agent, wherein the bioactive agent is associated via physicochemical interaction with a polymer, wherein the macrobead is obtainable by the method as described in claims 1 to 10.

15. A chitosan/alginate nanoparticles as defined in claim 13 or of an acid resistant alginate macrobead as defined in any of claims 14 for use in eliciting an immune response in an animal, wherein the animal is preferably a mammal or a bird.
